# Europäisches Patentamt
# European Patent Office
# Office européen des brevets

(19)

(11) Veröffentlichungsnummer: **0 079 461**
**B1**

(12) ## EUROPÄISCHE PATENTSCHRIFT

(45) Veröffentlichungstag der Patentschrift:
**05.12.84**

(21) Anmeldenummer: **82109323.4**

(22) Anmeldetag: **08.10.82**

(51) Int. Cl.³: **C 07 C  51/12,** C 07 C  51/56,
C 07 C  53/08, C 07 C  53/12,
C 07 C  67/36, C 07 C  69/16,
C 07 C  69/15, C 07 C  67/37,
C 07 C  69/14

(54) **Verfahren zur Herstellung von Essigsäureanhydrid und ggf. Essigsäure.**

(30) Priorität: **11.11.81  DE 3144772**

(43) Veröffentlichungstag der Anmeldung:
**25.05.83 Patentblatt 83/21**

(45) Bekanntmachung des Hinweises auf die Patenterteilung:
**05.12.84 Patentblatt 84/49**

(84) Benannte Vertragsstaaten:
**AT BE CH DE FR GB IT LI NL**

(56) Entgegenhaltungen:
**DE - A - 2 800 986**
**US - A - 4 102 921**

(73) Patentinhaber: **HOECHST AKTIENGESELLSCHAFT,
Postfach 80 03 20, D-6230 Frankfurt am Main 80 (DE)**

(72) Erfinder: **Vogt, Wilhelm, Dr., Bellerstrasse 74,
D-5030 Hürth (DE)**
Erfinder: **Glaser, Hermann, Magdalenenweg 16,
D-5042 Erftstadt (DE)**
Erfinder: **Koch, Jürgen, Dr., Am Römerkanal 10a,
D-5040 Brühl (DE)**

ACTORUM AG

## Beschreibung

Die Erfindung betrifft ein Verfahren zur Herstellung von Essigsäure und ggf. Essigsäureanhydrid durch Umsetzung von Methylacetat und/oder Dimethylether mit Kohlenmonoxid, ggf. in Gegenwart von Wasserstoff. Da mit Hilfe derartiger Carbonylierungsreaktionen $C_2$-Verbindungen auf Kohlebasis über Synthesegas als Zwischenstufe aufgebaut werden können, sind sie seit einigen Jahren Thema zahlreicher Patentanmeldungen. Zur Ausführung wird ein Katalysatorsystem benötigt, das häufig ein Edelmetall der Gruppe VIII des Periodensystems sowie Promotoren enthält. Als Promotoren werden meist zur Komplexbildung befähigte Amine oder Phosphine, ferner ein organisches Jodid oder Bromid sowie Nichtedelmetalle wie Chrom, Eisen, Kobalt oder Nickel genannt.

Es ist allerdings bemerkenswert, dass der aus mehreren bisher nicht geklärten Teilschritten bestehende Reaktionsablauf nur unter Verwendung von Jod- oder Bromverbindungen, besonders Methyljodid, als Promotor katalysiert werden konnte, so dass ein Carbonylierungsverfahren, wie es beispielsweise in der DE-A Nr. 2610036 beschrieben ist, aus wirtschaftlichen Gründen und auch wegen des Qualitätsstandards der Endprodukte einer sorgfältigen Wiedergewinnung der Jod- oder Bromverbindungen bedarf. Eine Möglichkeit dafür — Ausscheiden von Jod aus den Carbonylierungsprodukten bei 100 bis 175° C mit Cäsium-, Kalium- oder Natriumacetat — wurde in der DE-A Nr. 2940751 beschrieben. Es entstehen hierbei praktisch nichtflüchtige Alkalijodide. Der Fachmann musste daher annehmen, dass die wesentlich wohlfeileren Chlorverbindungen als Promotoren offenbar ungeeignet sind, und dass Alkalisalze die katalytische Wirkung jod- oder bromhaltiger Promotoren stören würden, da sie sie — zumindest innerhalb des angegebenen Temperaturbereiches von 100 bis 175° C — in Alkalijodide oder -bromide umwandeln.

Die Erfindung betrifft nun ein Verfahren zur Herstellung von Essigsäure und ggf. Essigsäureanhydrid durch Umsetzung von Methylacetat und/oder Dimethylether mit Kohlenmonoxid oder Gemischen von Kohlenmonoxid und Wasserstoff bei Temperaturen von 150 bis 300° C und Reaktionsdrucken oberhalb 80 bar in Gegenwart eines Katalysatorsystems aus Edelmetallen der Gruppe VIII des Periodensystems der Elemente oder deren Verbindungen sowie ggf. tertiären oder quaternären Organostickstoff- oder Organophosphorverbindungen, welches überraschenderweise dadurch gekennzeichnet ist, dass man zusätzlich als Promotor Methylchlorid, Chlor oder Chlorwasserstoff einsetzt.

Die Erfindung ist weiterhin wahlweise und bevorzugt dadurch gekennzeichnet, dass man

a) mit Methylchlorid oder Chlorwasserstoff quaternisierte Organostickstoff- oder Organophosphorverbindungen einsetzt;

b) als weiteren Promotor Alkaliacetat oder Alkaliverbindungen, die unter Reaktionsbedingungen zu Alkaliacetat umgewandelt werden, einsetzt;

c) Methylacetat oder Dimethylether/Edelmetall(verbindung)/Stickstoff- oder Phosphorverbindung/Chlor(verbindung)/Alkaliverbindung im Molverhältnis von 1:(0,0001 bis 0,01):(0,01 bis 1):(0,01 bis 1):(0 bis 0,1) einsetzt.

Als Nebenprodukt wird beim Verfahren der Erfindung vielfach gut verwertbares Ethylidendiacetat in geringeren Mengen erhalten.

Als Edelmetalle der Gruppe VIII des Periodensystems werden in erster Linie Rhodium, aber auch Palladium, Iridium oder Ruthenium eingesetzt. Man verwendet bevorzugt die Chloride oder Acetate, z.B. $RhCl_3 \cdot 3H_2O$, $IrCl_3$, $Pd(CH_3COO)_2$.

Als tertiäre Organostickstoff- oder Organophosphorverbindungen kommen Amine, Phosphine oder Aminophosphine in Frage, vorzugsweise Alkylamine, N-Alkylanilin, Pyridin, Pyrrolidone, Alkyl- oder Arylphosphine, insbesondere N-Methylimidazol, 3-Picolin, 2,4-Lutidin, 3,4-Lutidin, Chinolin, Tributylphosphin, Trioctylphosphin, Trilaurylphosphin oder Triphenylphosphin. Die Organostickstoff- oder Organophosphorverbindungen können jedoch auch quaternisiert mit Methylchlorid oder Chlorwasserstoff eingesetzt werden, z.B. als N-Methylpyridiniumchlorid, N,N-Dimethylimidazoliumchlorid, N-Methyl-3-picoliniumchlorid, N-Methyl-2,4-lutidiniumchlorid, N-Methyl-3,4-lutidiniumchlorid, N-Methylchinoliniumchlorid; Tributylmethylphosphoniumchlorid, Trioctylmethylphosphoniumchlorid, Trilaurylmethylphosphoniumchlorid, Triphenylmethylphosphoniumchlorid.

Die Zusammensetzung der Eingangsgase kann zwischen 100 Vol.-% CO und 20 Vol.-% CO+ 80 Vol.-% $H_2$ schwanken. Bevorzugt sind Volumenverhältnisse von CO:$H_2$=90:10 bis 50:50.

Vorzugsweise wird bei Temperaturen von 180 bis 250° C und Reaktionsdrucken von 100 bis 250 bar gearbeitet, doch sind auch Reaktionsdrucke von 300 bar und darüber möglich.

Die zur Reaktion eingesetzte Flüssigphase besteht im allgemeinen aus Methylacetat und/oder Dimethylether, Chlor(verbindung), einem zur Bildung von Carbonylverbindungen geeigneten Edelmetall der Gruppe VIII des Periodensystems der Elemente sowie einem Amin, Phosphin bzw. Aminophosphin, das in der Lage ist, das Absetzen unerwünschter Edelmetall enthaltender Niederschläge zu vermeiden. Dabei ist es gleichgültig, ob das Reaktionsgemisch das Katalysatorsystem gelöst enthält oder ob dieses unter Reaktionsbedingungen als zweite flüssige Phase vorliegt.

Die Reaktion wird im allgemeinen durch Zusatz kleiner Mengen Alkalisalze, insbesondere von Kalium, Rubidium und Cäsium beschleunigt; jedoch ist ihr Zusatz bei der Ausführung des Verfahrens unter technischen Bedingungen nicht zwingend erforderlich.

### Beispiel 1 (Vergleichsbeispiel)

In einem korrosionsfesten Edelstahlrührautoklav (1 l Inhalt) wurden 250 g Methylacetat und 1,6 g $RhCl_3 \cdot 3H_2O$ gefüllt. Nach Aufdrücken von 77 bar Kohlenmonoxid und 34 bar Wasserstoff wurde der Autoklav auf 240°C hochgeheizt, wobei sich ein Reaktionsdruck von 192 bar einstellte. Nach einer Verweilzeit von 5,3 h wurde abgekühlt, entspannt und der Autoklaveninhalt gaschromatographisch untersucht. Es konnten neben einer geringen Menge von 1,2 g Essigsäure keine Produktneubildungen festgestellt werden

*Beispiel 2:*

In den Rührautoklav aus Beispiel 1 wurden 250 g Methylacetat, 100 g Methylchlorid und 1,6 g $RhCl_3 \cdot 3H_2O$ eingefüllt. Nach dem Aufdrücken von 80 bar CO und 30 bar $H_2$ wurde der Autoklav 18 h auf 215°C erhitzt. Der Reaktionsdruck stieg bis auf 183ᐟ bar. Nach Aufarbeitung des Reaktionsgemisches wurde durch gaschromatographische Analyse eine Produktneubildung von 51,6 g Essigsäure, 14,1 g Essigsäureanhydrid und 0,2 g Ethylidendiacetat festgestellt.

*Beispiel 3:*

Neben 250 g Methylacetat, 100 g $CH_3Cl$, 1,6 g $RhCl_3 \cdot 3H_2O$ wurden noch 8,2 g N-Methylimidazol in den Autoklav gefüllt. Nach dem Aufdrücken von 80 bar CO und 30 bar $H_2$ wurde der Autoklav auf 220°C erhitzt, wobei sich ein Reaktionsdruck von 210 bar einstellte. Die Verweilzeit betrug 7 h. Nach Aufarbeitung konnte gaschromatographisch die Neubildung von 73,6 g Essigsäure und 44 g Essigsäureanhydrid ermittelt werden.

*Beispiel 4:*

In den Autoklav wurden 250 g Methylacetat, 100 g $CH_3Cl$, 50 g N-Methylimidazol und 1,6 g $RhCl_3 \cdot 3H_2O$ eingefüllt. Nach dem Aufdrücken von 80 bar CO und 30 bar $H_2$ konnten bei einer Reaktionstemperatur von 210°C und einer Verweilzeit von 5 h 66 g Essigsäure, 33 g Essigsäureanhydrid und 0,5 g Ethylidendiacetat erhalten werden.

*Beispiel 5:*

Auf ein Gemisch, bestehend aus 250 g Methylacetat, 100 g $CH_3Cl$, 13,3 g N,N-Dimethylimidazoliumchlorid, 1,6 g $RhCl_3 \cdot 3H_2O$, wurden 76 bar CO und 34 bar $H_2$ aufgedrückt. Nach einer Reaktionszeit von 4¼ h bei 220°C waren 38,1 g Essigsäure, 55,6 g Essigsäureanhydrid und 1,5 g Ethylidendiacetat entstanden.

*Beispiel 6:*

Das Reaktionsgemisch bestand aus 250 g Methylacetat, 26 g Methylchlorid, 13,3 g N,N-Dimethylimidazoliumchlorid, 1,6 g $RhCl_3 \cdot 3H_2O$. CO-Druck=76 bar, $H_2$-Druck=34 bar. Nach einer Reaktionszeit von 9½ h bei 240°C waren 76,1 g Essigsäure und 22,6 g Essigsäureanhydrid entstanden.

*Beispiel 7:*

Nach dem Aufdrücken von 83 bar CO, 38 bar $H_2$ auf ein Gemisch aus 250 g Methylacetat, 100 g Methylchlorid, 50 g N-Methylimidazol, 5 g Kaliumacetat, 1,6 g $RhCl_3 \cdot 3H_2O$ wurde der Autoklav 7 h auf 205°C erhitzt. Der Reaktionsdruck erreichte 195 bar. Erhalten wurden 108 g Essigsäure, 46 g Essigsäureanhydrid und 1 g Ethylidendiacetat.

*Beispiel 8:*

Nach dem Aufdrücken von 83 bar CO und 38 bar $H_2$ auf ein Gemisch von 250 g Methylacetat, 100 g Methylchlorid, 50 g N-Methylimidazol, 10 g Cäsiumacetat und 1,6 g $RhCl_3 \cdot 3H_2O$ wurden nach einer Reaktionszeit von 3 h bei 200°C, wobei der Reaktionsdruck 155 bar erreichte, 29 g Essigsäure, 41 g Essigsäureanhydrid und 0,2 g Ethylidendiacetat erhalten.

*Beispiel 9:*

Auf das Gemisch aus 250 g Methylacetat, 100 g Methylchlorid, 50 g N-Methylimidazol, 5 g Kaliumacetat, 1,35 g $Pd(CH_3COO)_2$ wurden 104 bar CO und 10 bar $H_2$ aufgedrückt. Nach einer Reaktionszeit von 8 h bei 240°C, wobei der Reaktionsdruck 191 bar erreichte, wurden 82 g Essigsäure und 7 g Essigsäureanhydrid erhalten.

*Beispiel 10:*

In den Autoklav wurden 250 g Methylacetat, 100 g Methylchlorid, 5 g Kaliumacetat und 1,4 g Ruthenium(III)chlorid eingefüllt. Nach Spülen mit CO-Gas wurden 80 bar CO und 20 bar $H_2$ aufgedrückt. Bei einer Temperatur von 210°C und einem maximalen Reaktionsdruck von 180 bar entstanden innerhalb von 5 h 30 g Essigsäure und 10 g Essigsäureanhydrid. Eine geringe Menge von 3,8 g Vinylacetat war zusätzlich entstanden.

*Beispiel 11:*

Auf eine Autoklavfüllung von 100 g Methylchlorid, 100 g Dimethylether, 50 g N-Methylimidazol und 1,6 g $RhCl_3 \cdot 3H_2O$ wurden 80 bar CO und 20 bar $H_2$ aufgedrückt. Bei einer Reaktionstemperatur von 200°C und einer Verweilzeit von 9 h konnten aus dem Reaktionsprodukt 53 g Methylacetat neben 36 g Essigsäure gewonnen werden.

## Patentansprüche

1. Verfahren zur Herstellung von Essigsäure und ggf. Essigsäureanhydrid durch Umsetzung von Methylacetat und/oder Dimethylether mit Kohlenmonoxid oder Gemischen von Kohlenmonoxid und Wasserstoff bei Temperaturen von 150 bis 300°C und Reaktionsdrücken oberhalb 80 bar in Gegenwart eines Katalysatorsystems aus Edelmetallen der Gruppe VIII des Periodensystems der Elemente oder deren Verbindungen sowie ggf. tertiären oder quaternären Organostickstoff- oder Organophosphorverbindungen, dadurch gekennzeichnet, dass man zusätzlich als Promotor Methylchlorid, Chlor oder Chlorwasserstoff einsetzt.

2. Verfahren nach Anspruch 1, dadurch gekennzeichnet, dass man mit Methylchlorid oder Chlorwasserstoff quaternisierte Organostickstoff- oder Organophosphorverbindungen einsetzt.

3. Verfahren nach einem der beiden Ansprüche 1 oder 2, dadurch gekennzeichnet, dass man als weiteren Promotor Alkaliacetat oder Alkaliverbindungen, die unter Reaktionsbedingungen zu Alkaliacetat umgewandelt werden, einsetzt.

4. Verfahren nach einem der Ansprüche 1 bis 3, dadurch gekennzeichnet, dass man Methylacetat oder Dimethylether/Edelmetall(verbindung)/ Stickstoff- oder Phosphorverbindung/Chlor(verbindung)/Alkaliverbindung im Molverhältnis von 1:(0,0001 bis 0,01):(0,01 bis 1):(0,01 bis 1): (0 bis 0,1) einsetzt.

## Claims

1. Process for making acetic acid and optionally acetic anhydride by reacting methyl acetate and/or dimethylether with carbon monoxide or mixtures of carbon monoxide and hydrogen, at temperatures of 150 to 300°C, under reaction pressures of more than 80 bar, in the presence of a catalyst system comprised of noble metals belonging to group VIII of the periodic system of the elements, or their compounds, and optionally tertiary or quaternary organonitrogen or organophosphorus compounds, which comprises using methyl chloride, chlorine or hydrogen chloride as an additional promoter.

2. Process as claimed in Claim 1, wherein organonitrogen or organophosphorus compounds quaternized with methyl chloride or hydrogen chloride are used.

3. Process as claimed in one of Claims 1 or 2, wherein alkali metal acetate or alkali metalcompounds transformed to alkali metal acetate under the reaction conditions are used as additional promoter.

4. Process as claimed in one of Claims 1 to 3, wherein methyl acetate or dimethylether/noble metal (compound)/nitrogen compound or phosphorus compound/chlorine(compound)/alkali metal compound are used in a molar ratio of 1/(0.0001-0.01)/(0.01-1)/(0.01-1)/(0-0.1).

## Revendications

1. Procédé de préparation de l'acide acétique et, éventuellement, de l'anhydride acétique par réaction de l'acétate de méthyle et/ou de l'éther diméthylique avec le monoxyde de carbone ou des mélanges monoxides de carbone/hydrogène à des températures de 150-300°C et sous des pressions de réaction supérieures à 80 bar en présence d'un système catalytique constitué par des métaux nobles appartenant au groupe VIII de la classification périodique des éléments ou leurs composés et, éventuellement, des composés organophosphorés ou organoazotés tertiaires ou quaternaires, caractérisé en ce que l'on utilise le chlorure de méthyle, le chlore ou le chlorure d'hydrogène comme promoteur supplémentaire.

2. Procédé selon la revendication 1, caractérisé en ce que l'on utilise des composés organophosphorés ou organoazotés quaternisés par du chlorure de méthyle ou du chlorure d'hydrogène.

3. Procédé selon l'une des revendications 1 ou 2, caractérisé en ce que l'on utilise, comme promoteur supplémentaire, un acétate alcalin ou des composés alcalins transformés en acétates alcalins dans les conditions de la réaction.

4. Procédé selon l'une des revendications 1 à 3, caractérisé en ce que l'on utilise: acétate de méthyle ou éther diméthylique/(composé de) métal noble/composé organophosphoré ou organoazoté/(composé du) chlore/composé alcalin dans des proportions molaires de: 1/(0,0001-0,01)/(0,01-1)/(0,01-1)/(0-0,1).